# EUROPEAN PATENT SPECIFICATION

(11) **EP 3 725 219 B1**
(45) Date of publication and mention of the grant of the patent: **22.01.2025**
(21) Application number: 20170120.8
(22) Date of filing: 17.04.2020
(51) Int. Cl.: A61B 5/00

(54) **FISH BIO SIGNAL MEASURING DEVICE**
VORRICHTUNG ZUR MESSUNG VON FISCHBIOSIGNALEN
DISPOSITIF DE MESURE DE SIGNAL BIOLOGIQUE DE POISSONS

(30) Priority: 17.04.2019 KR 20190044710
(43) Date of publication of application: 21.10.2020
(73) Proprietor: Daegu Gyeongbuk Institute Of Science and Technology, Daegu 42988 (KR)
(72) Inventor: KIM, So Hee, 42001 Daegu (KR); LEE, Yu Hyun, 47798 Busan (KR)
(74) Representative: Zimmermann, Tankred Klaus

(56) References cited:
- WO-A1-2005/117573
- CN-A- 105 962 927
- CN-A- 107 643 377
- JP-A- 2003 159 265

## Description

### BACKGROUND OF THE INVENTION

### Field of the Invention

The present invention relates to a bio signal measuring device of fish and, more particularly, to a bio signal measuring device of fish that can easily inject a necessary medicine into a plurality of fish and can easily fix the fish and electrodes.

### Description of the Related Art

In general, electroencephalography (EEG), electromyography (EMG) or electrocardiography (ECG), which is a test method that evaluates the function of brain, muscles or heart by recording activity potential that is generated when brain is active, muscle fibers or heart contract, performs tests by sensing an electrical change in brain, muscles or heart.

As muscles are controlled by nerves and a fine current always flows through muscles, it is possible to determine whether muscles normally act including even peripheral nerves by checking the current through a needle or an electrode and recording the current using an electromyography system.

Accordingly, EMG is usually used when there is a problem with a peripheral nerve and a muscle, and particularly, and is used to find out the state of muscles that weakens muscles such as muscle atrophy and nerve disorders.

As described above, a bio signal measurement technology is used in various fields such as not only EMG, but also electrocardiography (ECG) and electroencephalography (EEG) that is a brain wave measurement.

Document CN105962927 B discloses an on-line fish electrocardiogram collecting method and an on-line fish electrocardiogram-collecting device based on the swimming state. Therefore, two collection electrodes are buried near the heart to collect fish's ECG signals.

Document WO2005/11753 A1 describes an aquaculture system, relating to a system for aquaculture, to be used in environments where the aquaculture system is exposed to the elements such as in an open ocean environment.

Document JP2003159265 A is relating to an injection device for injecting a vaccine or the like into an injection body such as an animal, in particularly a fish.

Document CN107643377 A relates to a water-quality evaluation method and a system based on fish electrocardiogram indexes.

Recently, a technology that performs tests such as EMG, EEG, and ECG for a plurality of fish that have genetic information similar to that of human has been developed. For example, a system for performing EMG on Zebrafish, which is a vertebrate having genetic information similar to that of human, of a plurality of fish has been developed.

However, according to EMG systems for fish in the related art, it is difficult to simultaneously examine a plurality of fish and there is a trouble of inserting and fixing a fish into an agarose gel or a coil electrode.

Further, there are problems in the process of supplying medicines into a fish in the related art. The problems are described hereafter with reference to the following figure.

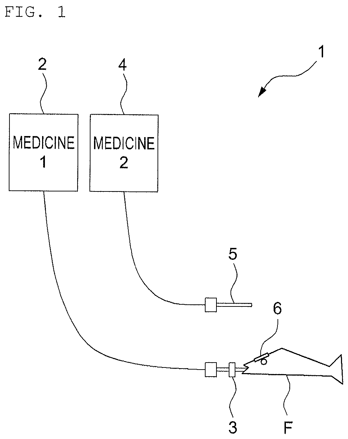

FIG. 1 is an exemplary view of a bio signal measuring device of fish according to the related art.

As shown in FIG. 1, a bio signal measuring device of fish 1 of the related art needs to change an oral needle every time to change a medicine. For example, in order to provide a first medicine 2 to a fish F, it is required to put a first oral needle 3 connected to the first medicine 2 into the mouth of the fish F and then measure a bio signal through an electrode 6. Thereafter, in order to provide a second medicine 4 to the fish F, it is required to pull out the first oral needle 3 and then connect the fish F to a second oral needle 5 connected to the second medicine 4.

Since it was required to separate and change the oral needle connected to the fish F every time to change a medicine in the related art, it was required to modify the circuit connected to the oral needle that was grounded. Further, when switching the oral needles, it was difficult to fix the fish F and the electrode because the fish F falls or the attached electrode is detached. Further, as it was required to increase the numbers of packs keeping medicines and oral needles in proportion to the number of fish F, the test environment was complicated, causing mistakes frequently.

### Citation List

### Patent Literature

Patent Literature 1: Japanese Patent Application Publication No. 2002-085362

### SUMMARY OF THE INVENTION

In order to solve the problems described above, an object of the present invention is to provide a bio signal measuring device of fish that can easily inject a necessary medicine into a plurality of fish and can easily fix the fish and electrodes during the change of medicines.

The objects to implement in the present invention are not limited to the technical problems described above and other objects that are not stated herein will be clearly understood by those skilled in the art from the following specifications.

In order to achieve the objects of the present invention, a bio signal measuring device of fish according to claim 1 is provided.

In an embodiment of the present invention, the fixing unit may have: a fixing main body forming a body; a fish seat formed on the top of the fixing main body so that the fish is fixed therein; and a needle seat formed in a hole shape, in which an oral needle may be inserted, in the front of the main body to communicate with the fish seat, and the fish seat may be formed in a groove shape corresponding to the shape of the fish so that the fish is fixed in an erected position.

In an embodiment of the present invention, the fixing unit may further have drains formed in both sides of the fixing main body, and the drain may discharge a medicine discharged from the gills of the fish to the outside from the main body.

In an embodiment of the present invention, the fixing unit may further have: a coupling groove formed at a side of the fixing main body; and a coupling protrusions formed at the other side of the fixing main body to be fitted in the coupling groove formed in another adjacent fixing main body, and the coupling groove and the coupling protrusion may maintain the gap between adjacent fixing main bodies.

In an embodiment of the present invention, the fixing unit may further have: a pad seat formed in the fixing main body in a groove shape that can fix an electrode pad of the electrode unit; and a wire hole formed in the rear of the main body to communicate with the pad seat, and the wire hole may be provided so that a wire connected to the electrode pad fixed in the pad seat is inserted therein.

In an embodiment of the present invention, the medicine storage unit may include a plurality of medicine storages keeping different medicines.

In an embodiment of the present invention, the bio signal measuring device of fish may further include a medicine valve unit disposed between the medicine storage unit and the medicine mixing unit, in which the medicine valve unit may include medicine valves disposed to correspond to the medicine storages, respectively, to control the amount of medicines to be supplied to the medicine mixing unit from the medicine storages and whether to supply medicines.

In an embodiment of the present invention, the medicine mixing unit may include: a medicine exchanger mixing the medicines provided from the medicine storage unit; and a plurality of medicine receivers disposed to correspond to the medicine storages, respectively, to provide the medicines in the medicine storages to the medicine exchanger.

In an embodiment of the present invention, the medicine distribution unit may include: a medicine distributor receiving the mixed medicine from the medicine mixing unit; and medicine distribution branches diverging from the medicine distributor to provide the mixed medicine to a plurality of fish, respectively.

In an embodiment of the present invention, the needle unit may be disposed between the medicine distribution unit and the fixing unit to correspond to the fixing unit.

In an embodiment of the present invention, the needle unit may include: an oral needle connected to the medicine distribution unit to receive a medicine and configured to be inserted into the mouth of the fish to inject a medicine; and a needle holder fixing the oral needle.

In an embodiment of the present invention, the needle holder may adjust a height and an insertion depth of the oral needle.

In an embodiment of the present invention, the bio signal measuring device of fish may further include a ground unit fixed to the oral needle.

In an embodiment of the present invention, the electrode unit may include: an electrode pad fixed to the fixing unit; and an electrode connected to the electrode pad to measure a bio signal of the fish.

### BRIEF DESCRIPTION OF THE DRAWINGS

FIG. 1 is an exemplary view of a bio signal measuring device of fish according to the related art;
FIG. 2 is a picture of a bio signal measuring device of fish according to the present invention;
FIGS. 3 and 4 are perspective views of a fixing unit of the bio signal measuring device of fish according to an embodiment of the present invention;
FIG. 5 is a top view of the fixing unit of the bio signal measuring device of fish according to an embodiment of the present invention;
FIG. 6 is a side view of the fixing unit of the bio signal measuring device of fish according to an embodiment of the present invention;
FIG. 7 is an exemplary view showing the configuration of the bio signal measuring device of fish according to an embodiment of the present invention;
FIG. 8 is a picture of the fixing unit of the bio signal measuring device of fish according to an embodiment of the present invention;
FIG. 9 is a perspective view showing a lower needle holder of a needle unit of the bio signal measuring device according to an embodiment of the present invention;
FIG. 10 is a perspective view showing an upper needle holder of the needle unit of the bio signal measuring device according to an embodiment of the present invention; and
FIG. 11 is a top view showing the assembly of the fixing unit and the needle unit of the bio signal measuring device according to an embodiment of the present invention.

### DETAILED DESCRIPTION OF THE PREFERRED EMBODIMENTS

Hereinafter, the present invention is described with reference to the accompanying drawings. However, the present invention may be modified in various different ways and is not limited to the embodiments described herein. Further, in the accompanying drawings, components irrelevant to the description will be omitted in order to obviously describe the present invention, and similar reference numerals will be used to describe similar components throughout the specification.

Throughout the specification, when an element is referred to as being "connected with (coupled to, combined with, in contact with)" another element, it may be "directly connected" to the other element and may also be "indirectly connected" to the other element with another element intervening therebetween. Further, unless explicitly described otherwise, "comprising" any components will be understood to imply the inclusion of other components rather than the exclusion of any other components.

Terms used in the present invention are used only in order to describe specific exemplary embodiments rather than limiting the present invention. Singular forms are intended to include plural forms unless the context clearly indicates otherwise. It will be further understood that the terms "comprises" or "have" used in this specification, specify the presence of stated features, steps, operations, components, parts, or a combination thereof, but do not preclude the presence or addition of one or more other features, numerals, steps, operations, components, parts, or a combination thereof.

Hereinafter, embodiments are described in detail with reference to the accompanying drawings.

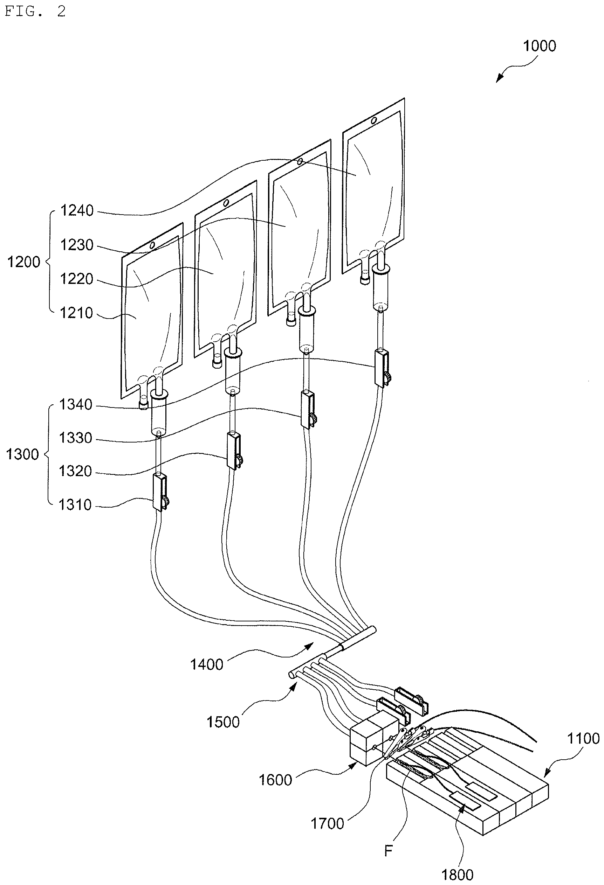

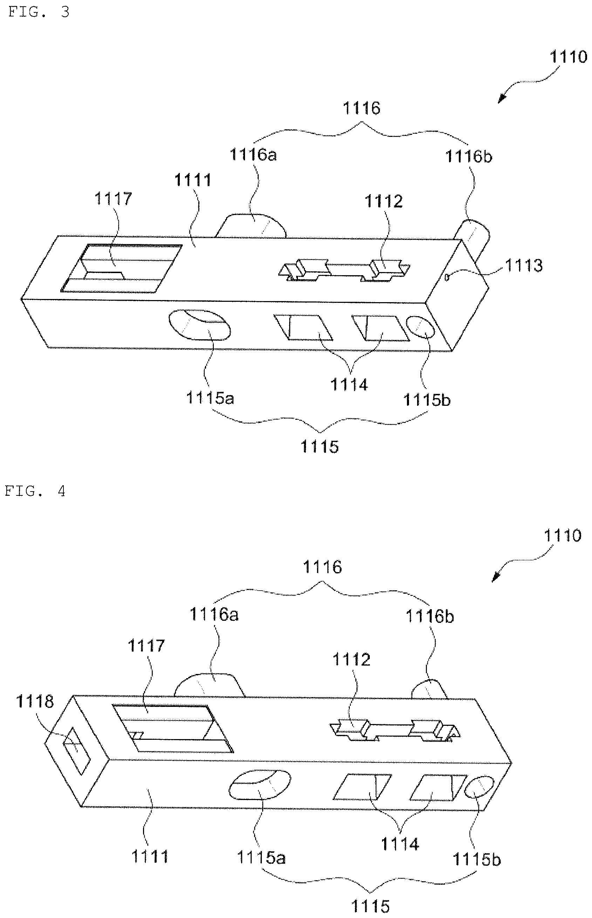

FIG. 2 is a picture of a bio signal measuring device of fish according to the present invention, and FIGS. 3 and 4 are perspective views of a fixing unit of the bio signal measuring device of fish according to an embodiment of the present invention.

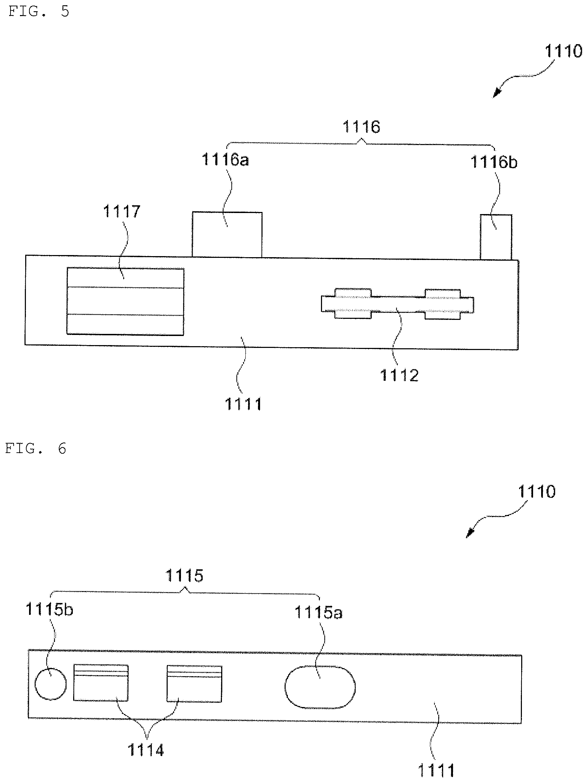

FIG. 5 is a plan view of the fixing unit of the bio signal measuring device of fish according to an embodiment of the present invention, and FIG. 6 is a side view of the fixing unit of the bio signal measuring device of fish according to an embodiment of the present invention.

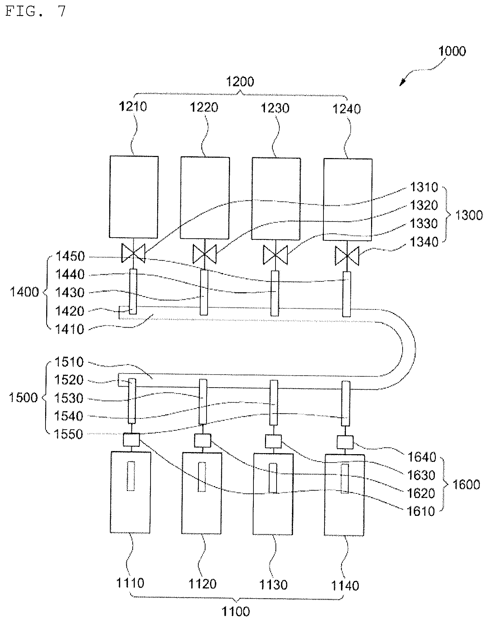

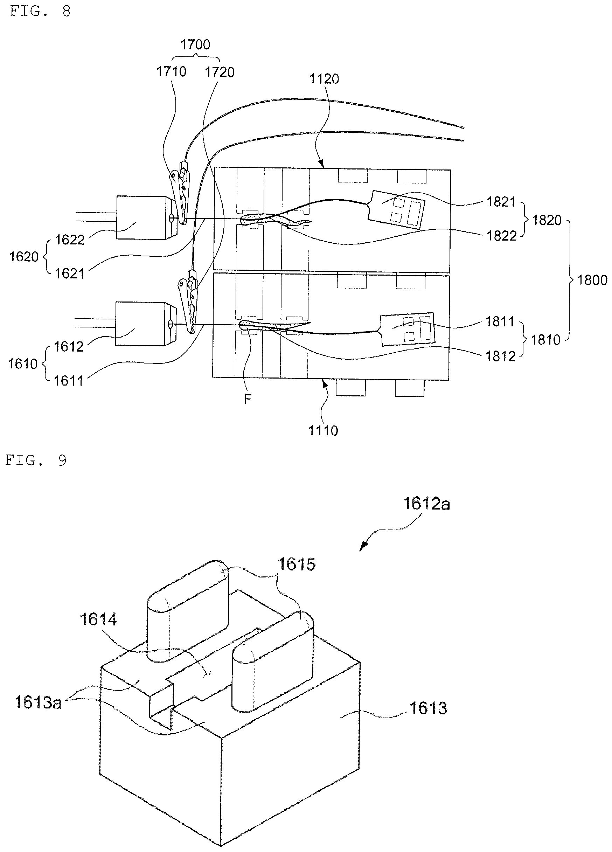

FIG. 7 is an exemplary view showing the configuration of the bio signal measuring device of fish according to an embodiment of the present invention, and FIG. 8 is a picture of the fixing unit of the bio signal measuring device of fish according to an embodiment of the present invention.

As shown in FIGS. 2 to 8, a bio signal measuring device of fish 1000 includes a fixing unit 1100, a medicine storage unit 1200, a medicine valve unit 1300, a medicine mixing unit 1400, a medicine distribution unit 1500, a needle unit 1600, a ground unit 1700, and an electrode unit 1800.

The fixing unit 1100 is provided to fix fish and may be provided in several pieces. Although a first fixing unit 1110, a second fixing unit 1120, a third fixing unit 1130 and a fourth fixing unit 1140 are shown in the figures of the present invention, the number of the fixing units is not limited thereto. That is, a plurality of fixing units 1110, 1120, 1130, 1140 may be provided and connected to each other in parallel, and the number of the fixing units 1110, 1120, 1130, 1140 may be increased or decreased, if necessary, to simultaneously examine a plurality of fish F.

Hereafter, the configuration of the fixing unit 1100 is described in detail on the basis of the first fixing unit 1110 of the bio signal measuring device of the present invention.

The first fixing unit 1110 have a fixing main body 1111, a fish seat 1112, a needle seat 1113, a drain 1114, a first coupling groove 1115a, a second coupling groove 1115b, a first coupling protrusion 1116a, a second coupling protrusion 1116b, a pad seat 1117, and a wire hole 1118.

The fixing main body 1111 may form the body of the first fixing unit 1110. The fixing main body 1111, as shown in the figures, may have a rectangular parallelepiped shape, but is not limited thereto.

The fish seat 1112 may be formed on the top of the fixing main body 1111 so that the fish F can be fixed therein. In detail, the fish seat 1112 may be provided by forming a groove in a shape corresponding to the shape of the fish F so that the fish F can be fixed in an erect position. Here, the fish F may be Zebrafish.

The needle seat 1113 may be formed in a hole shape, in which an oral needle of the needle unit 1600 can be inserted, in the front of the fixing main body 1111 to communicate with the fish seat 1112.

That is, the needle seat 1113 may be provided so that the oral needle inserted through the needle seat 1113 is inserted into the mouth of a fish fixed in the fish seat 1112.

The drain 1114 is formed at both sides of the fixing main body 1111 to discharge the medicine discharged from the gills of the fish F to the outside from the fixing main body 1111.

The first coupling groove 1115a and the second coupling groove 1115b may be formed at a side of the fixing main body 1111.

The first coupling protrusion 1116a and the second coupling protrusion 1116b may be formed at the other side of the fixing main body 1111 to be fitted in the coupling grooves formed in another adjacent fixing main body.

In detail, the first coupling protrusion 1116a may be formed in a shape corresponding to the first coupling groove 1115a and the second coupling protrusion 1116b may be formed in a shape corresponding to the second coupling groove 1115b. For example, the first coupling protrusion 1116a of the first fixing unit 1110 is fitted in the first coupling groove of the adjacent second fixing unit 1120 and the second coupling protrusion 1116b is fitted in the second coupling groove of the second fixing unit 1120, whereby the first fixing unit 1110 and the second fixing unit 1120 can be combined.

The coupling grooves and the coupling protrusions provided as described above can maintain the gap between adjacent fixing main bodies.

The pad seat 1117 is formed in the fixing main body 1111 in a groove shape that can fix a first electrode pad 1811 of the electrode unit 1800.

The wire hole 1118 may be formed in the rear of the fixing main body 1111 to communicate with the pad seat 1117. The wire hole 1118 provided as described above may be provided so that a wire connected to the first electrode pad 1811 fixed in the pad seat 1117 is inserted therein.

The medicine storage unit 1200 can keep medicines to be supplied to the fish F fixed on the fixing unit 1100.

The medicine storage unit 1200 may have a plurality of medicine storages keeping different medicines. For example, as shown in the figures, the medicine storage unit 1200 may include a first medicine storage 1210, a second medicine storage 1220, a third medicine storage 1230, and a fourth medicine storage 1240, and different medicines can be kept in the respective medicine storages. However, the number of the medicine storages is not limited thereto.

Dissolved oxygen may be further included in the medicine kept in the medicine storage unit 1200 so that the fish F can be kept alive by breathing even while bio signals of the fish F are measured.

For example, any one of the medicine storages of the medicine storage unit 1200 keeps liquid containing dissolved oxygen to be able to keep supplying dissolved oxygen even while a medicine is not supplied to the fish F. The medicine storage unit 1200 having this configuration can enable the fish F to keep breathing and alive when an examination is in progress or even when not.

The present invention described above can measure more accurate bio signals in comparison to measuring bio signals of a dead fish or dying fish by enabling the fish F to keep breathing.

The medicine valve unit 1300 may be disposed between the medicine storage unit 1200 and the medicine mixing unit 1400. The medicine valve unit 1300 may be provided to correspond to the medicine storages one to one, thereby controlling the amount of medicines to be supplied to the medicine mixing unit 1400 from the medicine storages and whether to supply medicines.

For example, a first medicine valve 1310 may be disposed between the first medicine storage 1210 and the medicine mixing unit 1400 to control the amount of medicine to be supplied to the medicine mixing unit 1400 from the first medicine storage 1210 and whether to supply a medicine.

A second medicine valve 1320, a third medicine valve 1330, and a fourth medicine valve 1340 may also be connected to the second medicine storage 1220, the third medicine storage 1230, and the fourth medicine storage 1240, respectively, to perform the same function as the first medicine valve 1310.

The medicine valve unit 1300 having this configuration can make various medicines be more easily mixed by selectively opening/closing the medicine valves.

The medicine mixing unit 1400 is provided to mix the medicines provided from the medicine storage unit 1200 and includes a medicine exchanger 1410, a first medicine receiver 1420, a second medicine receiver 1430, a third medicine receiver 1440, and a fourth medicine receiver 1450.

The medicine exchanger 1410 may be provided to mix the medicines provided from the medicine storage unit 1200.

The medicine receivers are provided to correspond to the medicine storage one to one to provide the medicines in the medicine storages to the medicine exchanger 1410.

For example, the first medicine receiver 1420 is connected to the first medicine storage 1210, thereby being able to provide the medicine provided from the first medicine storage 1210 to the medicine exchanger 1410.

The second medicine receiver 1430, the third medicine receiver 1440, and the fourth medicine receiver 1450 may also be connected to the second medicine storage 1220, the third medicine storage 1230, and the fourth medicine storage 1240, respectively, to perform the same function as the first medicine receiver 1420.

The medicine distribution unit 1500 is provided to receive the mixed medicine from the medicine mixing unit 1400 and distribute the mixed medicine to a plurality of fish F, and may include a medicine distributor 1510, a first medicine distribution branch 1520, a second medicine distribution branch 1530, a third medicine distribution branch 1540, and a fourth medicine distribution branch 1550.

The medicine distributor 1510 can be provided with the mixed medicine from the medicine mixing unit 1400.

The first medicine distribution branch 1520, the second medicine distribution branch 1530, the third medicine distribution branch 1540, the fourth medicine distribution branch 1540 may diverge from the medicine distributor 1510 to provide the mixed medicine to a plurality of fish F.

The needle unit 1600 may be provided to inject the medicines distributed from the medicine distribution unit 1500 to a plurality of fish F, respectively. The needle unit 1600 is disposed between the medicine distribution unit 1500 and the fixing unit 1100 and may be provided as several pieces to correspond to the fixing units 1100 one to one.

For example, a first needle unit 1610 may be disposed between the first medicine distribution branch 1520 and the fixing unit 1100 to inject a medicine into the fish F fixed on the first fixing unit 1110.

A second needle unit 1620, a third needle unit 1630, and a fourth needle unit 1640 may also be connected to the second fixing unit 1120, the third fixing unit 1130, and the fourth fixing unit 1140, respectively, to perform the same function as the first needle unit 1610.

In detail, the first needle unit 1610 may include a first oral needle 1611 and a first needle holder 1612.

The first oral needle 1611 is connected to the medicine distribution unit 1500 and provided with a medicine, and is inserted into the mouth of the fish F to inject the medicine into the fish F.

The first needle holder 1612 may fix the first oral needle 1611. The first needle holder 1612 may be provided to adjust the height of the fixed first oral needle 1611.

The first needle holder 1612 may be provided to adjust the position of the first needle 1611 in accordance with the distance from the mouth of the fish F.

The configuration of the first needle holder 1612 is described in detail with reference to the following figures.

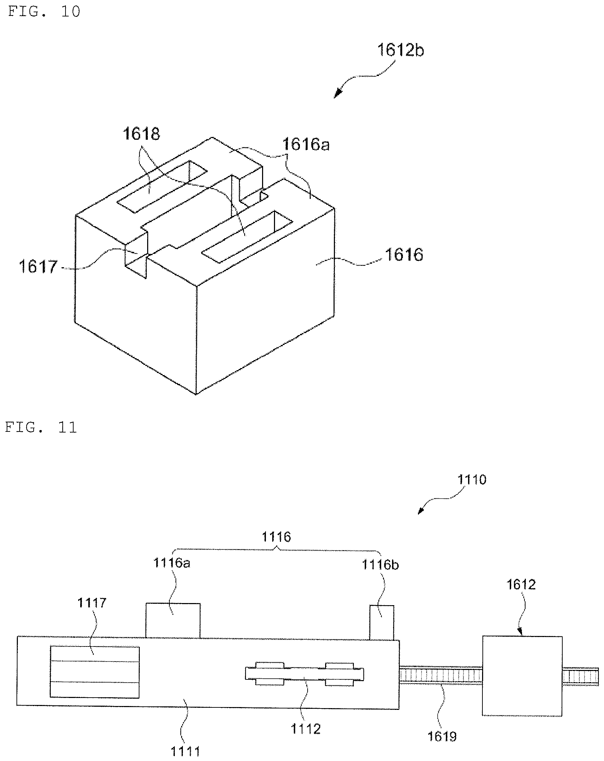

FIG. 9 is a perspective view showing a lower needle holder of a needle unit of the bio signal measuring device according to an embodiment of the present invention, FIG. 10 is a perspective view showing an upper needle holder of the needle unit of the bio signal measuring device according to an embodiment of the present invention, and FIG. 11 is a plan view showing the assembly of the fixing unit and the needle unit of the bio signal measuring device according to an embodiment of the present invention.

As shown in FIGS. 8 to 11, the first needle holder 1612 may be formed by combining a lower needle holder 1612a and an upper needle holder 1612b.

The lower needle holder 1612a may have a lower fixing body 1613, lower fixing walls 1613a, a lower needle seat groove 1614, lower couplers 1615.

The lower fixing body 1613 forms the body of the lower needle holder 1612a and may have a cuboid shape. However, the shape of the lower fixing body 1613 is not limited thereto.

The lower fixing walls 1613a are disposed at the upper portion of the lower fixing body 1613 and may extend toward the upper portion of the lower fixing body 1613. The lower fixing walls 1613a may be provided in a pair and spaced apart from each other at both sides of the upper portion of the lower fixing body 1613.

The lower needle seat groove 1614 in which the first oral needle 1611 can be seated may be formed between the pair of lower fixing walls 1613a.

The lower coupler 1615 may extend toward the upper portion from the lower fixing walls 1613a.

The upper needle holder 1612b may have an upper fixing body 1616, upper fixing walls 1616a, an upper needle seat groove 1617, and upper coupling grooves 1618.

The upper fixing body 1616 forms the body of the upper needle holder 1612b and may have a cuboid shape. However, the shape of the upper needle holder 1616 is not limited thereto.

The upper fixing walls 1616a are disposed at the upper portion of the upper fixing body 1616 and may extend toward the upper portion of the upper fixing body 1616. The upper fixing walls 1616a may be provided in a pair and spaced apart from each other at both sides of the upper portion of the upper fixing body 1616.

The upper fixing walls 1616a may be formed to correspond to the lower fixing walls 1613a so that the upper and lower fixing walls can be brought in contact with each other.

The upper needle seat groove 1617 in which the first oral needle 1611 can be seated may be formed between the pair of upper fixing walls 1616a. That is, the first oral needle 1611 may be inserted and fixed in the lower needle seat groove 1614 and the upper needle seat groove 1617.

The upper coupling grooves 1618 may be formed in groove shapes in the upper fixing walls 1616a. The upper coupling grooves 1618 may be formed in shapes at positions that enables the lower couplers 1615 to be inserted and fixed therein. That is, the lower needle holder 1612a and the upper needle holder 1612b can be combined by coupling the lower couplers 1615 and the upper coupling grooves 1618. In this case, the first oral needle 1611 may be inserted and fixed in the lower needle seat groove 1614 and the upper needle seat groove 1617.

The first needle holder 1612 may further have a guide rail 1619.

The guide rail 1619 may be coupled to connect the first needle holder 1612 and the fixing main body 1111 of the fixing unit 1110. The guide rail 1619 can enable the first oral needle 1611 coupled to the first needle holder 1612 to move only straight at a height and a position that correspond to the needle seat 1113.

In detail, when inserting the first oral needle 1611 fixed to the first needle holder 1612 into the needle seat 1113, it is difficult to insert the first oral needle 1611 into the small needle seat 1113.

However, when the guide rail 1619 is used, the first needle holder 1612 moves straight in the longitudinal direction of the guide rail 1619 without laterally moving, so it is possible to easily adjust the insertion depth of the first oral needle 1611 in the needle seat 1113.

Further, though not shown, the first needle holder 1612 may further have a movement control mechanism that limits the movement distance when moving on the guide rail 1619.

In detail, the movement control mechanism can allow the first needle holder 1612 to move a predetermined distance at a time and can fix the first needle holder 1612 at the position when there is no additional operation. For example, a control mechanism having teeth may be disposed between the lower needle holder 1612a and the guide rail 1619 so that the lower needle holder 1612a moves only by one step in accordance with the shape of the teeth every time it moves.

The movement control mechanism can make it easy to adjust the insertion depth of the first oral needle 1611 in the mouth of the fish F.

The first needle holder 1612 having the configuration describe above can allow for a stable examination by fixing the first oral needle 1611 inserted in the fish F at appropriate height and depth.

Further, according to the present invention, the first needle unit 1610 is fixed without a need for replacement and change even when the medicine storage unit 1200, the medicine valve unit 1300, the medicine mixing unit 1400, and the medicine distribution unit 1500 are changed, so it is convenient.

The second needle unit 1620 may also include a second oral needle 1621 and a second needle holder 1622 and the function thereof is the same as that of the first needle unit 1610.

The ground unit 1700 can be grounded by being fixed to the oral needle. In detail, the first ground unit 1710 is fixed to the first oral needle 1611 and is grounded, and the first oral needle 1611, as described above, is fixed without a need for replacement even when a medicine is changed. Accordingly, there is no possibility of the mistake that a user forgets grounding after replacing an oral needle when replacing a medicine, so examinations can be stably performed.

The second ground unit 1720 can be fixed to the second oral needle 1621 and can be grounded. As described above, the ground unit 1700 may be provided and fixed to correspond to each of the oral needles one to one.

The electrode unit 1800 is configured to be attached to the fish F to measure bio signals of the fish F and may correspond to the fixing unit 1100 one to one.

For example, the electrode unit 1800 may include a first electrode unit 1810, a first electrode pad 1811, and a first electrode 1812.

The first electrode pad 1811 may be fixed to the first fixing unit 1110. In detail, the first electrode pad 1811 may be seated in the pad seat 1117 of the first fixing unit 1110. A wire extending downward from the first electrode pad 1811 may extend outside through the wire hole 1118 from the pad seat 1117 to be connected.

The first electrode 1812 may be connected to the first electrode pad 1811 and the fish F to measure bio signals of the fish F. In this state, since the fish F is fixed in the fish seat 1112, it is possible to easily fix the first electrode 1812 and measure bio signals.

The second electrode unit 1820 may also include a second electrode pad 1821 and a second electrode 1822 to perform the same function as the first electrode unit 1810.

As described above, since the present invention measures bio signals while keeping a fish F alive, it is possible to obtain an accurate examination result and it is possible to simultaneously measure bio signals with several fish F fixed.

Further, it was required in the related art to replace oral needles to replace medicines and modify the circuit grounded for the oral needles. Further, there was the inconvenience that a fish falls down or an electrode is separated when an oral needle is replaced. However, according to the present invention, since it is possible to replace medicines without changing the fixing unit 1100, the needle unit 1600, and the ground unit 1700, a quick examination is possible and the mistake of non-grounding can be prevented.

Further, in the related art, when the number of fish was increased, it was required to increase the numbers liquid packs and oral needles in proportion to the number of fish, so the examination environment was complicated and there were frequent mistakes. However, according to the present invention, there is no need for increasing the number of the medicine storages in correspondence to the number of fish F, so medicine can be saved, which is economical.

An effect of the present invention having the configuration described above is to be able to simultaneously measure bio signals of several fish.

It is not required to replace the oral needles every time to replace medicines and there is no possibility of making a mistake of not connecting the ground unit.

Since a medicine kept in one medicine storage is shared by several fish, it is possible to reduce consumption of medicines, which is economical.

The effects of the present invention are not limited thereto and it should be understood that the effects include all effects that can be inferred from the configuration of the present invention described in the following specification or claims.

The above description is provided as an exemplary embodiment of the present invention and it should be understood that the present invention may be easily modified in other various ways within the scope of the claims.

Therefore, the embodiments described above are only examples and should not be construed as being limitative in all respects. For example, the components described as single parts may be divided and the components described as separate parts may be integrated.

The scope of the present invention is defined by the following claims.

## Claims

1. A bio signal measuring device (1000) for measuring bio signals of fish comprising:
a fixing unit (1100) for fixing fish, wherein the fixing unit (1100) comprises a fish seat (1112) having a groove corresponding to the shape of the fish so that the fish is fixed therein;
an electrode unit (1800) configured to be attached to the fish to measure a bio signal of the fish; **characterized in that** the bio signal measuring device further comprises:
a medicine storage unit (1200) for storing medicines to be supplied to the fish fixed on the fixing unit (1100), wherein the medicine storage unit (1200) includes a plurality of medicine storages for storing different medicines, and one of the medicine storages keeps liquid containing dissolved oxygen to be supplied to the fish so that the fish is enabled to keep breathing and alive when an examination is in progress or even when not;
a medicine mixing unit (1400) for mixing the medicines provided from the medicine storage unit (1200);
a medicine distribution unit (1500) for receiving the mixed medicines from the medicine mixing unit (1400) and distributing the mixed medicines to each of a plurality of fish;
a needle unit (1600) injecting the medicines distributed from the medicine distribution unit (1500) to each fish, wherein the needle unit (1600) comprises an oral needle connected to the medicine distribution unit (1500) to receive the medicine and configured to be inserted into the mouth of the fish to inject the medicines.

2. The bio signal measuring device (1000) of claim 1, wherein the fixing unit (1100) has:
a fixing main body (1111) forming a body;
and
a needle seat (1113) formed in a hole shape, in which the oral needle can be inserted, in the front of the fixing main body (1111) to communicate with the fish seat (1112), and
wherein the fish seat (1112) is formed on the top of the fixing main body (1111) and the fish is fixed in an erected position in the groove.

3. The bio signal measuring device (1000) of claim 2, wherein the fixing unit (1100) further has drains (1114) formed at both sides of the fixing main body (1111), and
the drains (1114) discharges medicines discharged from the gills of the fish to the outside from the fixing main body (1111).

4. The bio signal measuring device (1000) of claim 2, wherein the fixing unit (1100) further has:
a coupling groove formed at a side of the fixing main body (1111); and
a coupling protrusion formed at the other side of the fixing main body (1111) to be fitted in the coupling groove formed in another adjacent fixing main body (1111), and
the coupling groove and the coupling protrusion maintain the gap between adjacent fixing main bodies.

5. The bio signal measuring device (1000) of claim 2, wherein the fixing unit (1100) further has:
a pad seat (1117) formed in the fixing main body (1111) in a groove shape that can fix electrode pads (1811, 1821) of the electrode unit (1800); and
a wire hole (1118) formed in the rear of the fixing main body (1111) to communicate with the pad seat (1117), and
the wire hole (1118) is provided so that a wire connected to the electrode pads (1811, 1821) fixed in the pad seat (1117) is inserted therein.

6. The bio signal measuring device (1000) of claim 1, further comprising a medicine valve unit (1300) disposed between the medicine storage unit (1200) and the medicine mixing unit (1400),
wherein the medicine valve unit (1300) includes medicine valves disposed to correspond to the medicine storages, respectively, to control the amount of medicines to be supplied to the medicine mixing unit (1400) from the medicine storages and whether to supply medicines.

7. The bio signal measuring device (1000) of claim 1, wherein the medicine mixing unit (1400) includes:
a medicine exchanger (1410) mixing the medicines provided from the medicine storage unit (1200); and
a plurality of medicine receivers disposed to correspond to the medicine storages, respectively, to provide the medicines in the medicine storages to the medicine exchanger (1410).

8. The bio signal measuring device (1000) of claim 1, wherein the medicine distribution unit (1500) includes:
a medicine distributor (1510) for receiving the mixed medicine from the medicine mixing unit (1400); and
medicine distribution branches diverging from the medicine distributor (1510) to provide the mixed medicine to the plurality of fish, respectively.

9. The bio signal measuring device (1000) of claim 1, wherein the needle unit (1600) is disposed between the medicine distribution unit (1500) and the fixing unit (1100) to correspond to the fixing unit (1100) one to one.

10. The bio signal measuring device (1000) of claim 9, wherein the needle unit (1600) includes:
a needle holder fixing the oral needle.

11. The bio signal measuring device (1000) of claim 10, wherein the needle holder can adjust a height and an insertion depth of the oral needle.

12. The bio signal measuring device (1000) of claim 1, further comprising a ground unit (1700) fixed to the oral needle.

13. The bio signal measuring device (1000) of claim 1, wherein the electrode unit (1800) includes:
electrode pads (1811, 1821) fixed to the fixing unit (1100); and
electrodes (1812, 1822) connected to the electrode pads (1811, 1821) to measure a bio signal of the fish.

## Patentansprüche

1. Biosignalmessvorrichtung (1000) zum Messen von Biosignalen von Fischen, die folgende Merkmale aufweist:
eine Fixiereinheit (1100) zum Fixieren von Fischen, wobei die Fixiereinheit (1100) eine Fischauflage (1112) mit einer Rille aufweist, die der Form des Fisches entspricht, so dass der Fisch darin fixiert ist;
eine Elektrodeneinheit (1800), die dazu konfiguriert ist, an dem Fisch angebracht zu werden, um ein Biosignal des Fisches zu messen; **dadurch gekennzeichnet, dass** die Biosignalmessvorrichtung ferner folgende Merkmale aufweist:
eine Arzneimittelspeichereinheit (1200) zum Speichern von Arzneimitteln, die dem an der Fixiereinheit (1100) fixierten Fisch verabreicht werden sollen, wobei die Arzneimittelspeichereinheit (1200) eine Mehrzahl von Arzneimittelspeichern zum Speichern unterschiedlicher Arzneimittel aufweist und einer der Arzneimittelspeicher Flüssigkeit hält, die gelösten Sauerstoff enthält, der dem Fisch verabreicht werden soll, so dass der Fisch weiter atmen und am Leben gehalten werden kann, wenn eine Untersuchung im Gange ist oder auch, wenn dies nicht der Fall ist;
eine Arzneimittelmischeinheit (1400) zum Mischen der Arzneimittel, die von der Arzneimittelspeichereinheit (1200) bereitgestellt werden;
eine Arzneimittelverteilungseinheit (1500) zum Aufnehmen der gemischten Arzneimittel von der Arzneimittelmischeinheit (1400) und Verteilen der gemischten Arzneimittel an jeden einer Mehrzahl von Fischen;
eine Nadeleinheit (1600), die die von der Arzneimittelverteilungseinheit (1500) verteilten Arzneimittel jedem Fisch injiziert, wobei die Nadeleinheit (1600) eine Oralnadel aufweist, die mit der Arzneimittelverteilungseinheit (1500) verbunden ist, um das Arzneimittel aufzunehmen, und dazu konfiguriert ist, in den Mund des Fisches eingeführt zu werden, um die Arzneimittel zu injizieren.

2. Biosignalmessvorrichtung (1000) gemäß Anspruch 1, bei der die Fixiereinheit (1100) folgende Merkmale aufweist:
einen Fixierhauptkörper (1111), der einen Körper bildet; und
eine Nadelauflage (1113), die in einer Lochform gebildet ist, in die die Oralnadel eingeführt werden kann, an der Vorderseite des Fixierhauptkörpers (1111), um mit der Fischauflage (1112) in Verbindung zu stehen, und
wobei die Fischauflage (1112) an der Oberseite des Fixierhauptkörpers (1111) gebildet ist und der Fisch in einer aufgerichteten Position in der Rille fixiert ist.

3. Biosignalmessvorrichtung (1000) gemäß Anspruch 2, bei der die Fixiereinheit (1100) ferner Abläufe (1114) aufweist, die an beiden Seiten des Fixierhauptkörpers (1111) gebildet sind, und
die Abläufe (1114) Arzneimittel, die von den Kiemen des Fisches abgegeben werden, von dem Fixierhauptkörper (1111) nach außen abgeben.

4. Biosignalmessvorrichtung (1000) gemäß Anspruch 2, bei der die Fixiereinheit (1100) ferner folgende Merkmale aufweist:
eine Kopplungsrille, die an einer Seite des Fixierhauptkörpers (1111) gebildet ist; und
einen Kopplungsvorsprung, der an der anderen Seite des Fixierhauptkörpers (1111) gebildet ist, um in die Kopplungsrille eingepasst zu werden, die in einem anderen benachbarten Fixierhauptkörper (1111) gebildet ist, und
die Kopplungsrille und der Kopplungsvorsprung den Spalt zwischen benachbarten Fixierhauptkörpern aufrechterhalten.

5. Biosignalmessvorrichtung (1000) gemäß Anspruch 2, bei der die Fixiereinheit (1100) ferner folgende Merkmale aufweist:
eine Anschlussflächenauflage (1117), die in dem Fixierhauptkörper (1111) in einer Rillenform gebildet ist, die Elektrodenanaschlussflächen (1811, 1821) der Elektrodeneinheit (1800) fixieren kann; und
ein Drahtloch (1118), das an der Rückseite des Fixierhauptkörpers (1111) gebildet ist, um mit der Anschlussflächenauflage (1117) in Verbindung zu stehen, und
das Drahtloch (1118) so vorgesehen ist, dass ein Draht, der mit den Elektrodenanschlussflächen (1811, 1821) verbunden ist, die in der Anschlussflächenauflage (1117) fixiert sind, in dasselbe eingeführt wird.

6. Biosignalmessvorrichtung (1000) gemäß Anspruch 1, die ferner eine Arzneimittelventileinheit (1300) aufweist, die zwischen der Arzneimittelspeichereinheit (1200) und der Arzneimittelmischeinheit (1400) angeordnet ist, wobei die Arzneimittelventileinheit (1300) Arzneimittelventile aufweist, die so angeordnet sind, dass sie jeweils den Arzneimittelspeichern entsprechen, um die Menge an Arzneimitteln zu steuern, die der Arzneimittelmischeinheit (1400) aus den Arzneimittelspeichern zugeführt werden sollen, und zu steuern, ob Arzneimittel zugeführt werden sollen.

7. Biosignalmessvorrichtung (1000) gemäß Anspruch 1, bei der die Arzneimittelmischeinheit (1400) folgende Merkmale aufweist:
einen Arzneimitteltauscher (1410), der die Arzneimittel mischt, die von der Arzneimittelspeichereinheit (1200) bereitgestellt werden; und
eine Mehrzahl von Arzneimittelaufnehmern, die so angeordnet sind, dass sie jeweils den Arzneimittelspeichern entsprechen, um die Arzneimittel in den Arzneimittelspeichern für den Arzneimitteltauscher (1410) bereitzustellen.

8. Biosignalmessvorrichtung (1000) gemäß Anspruch 1, bei der die Arzneimittelverteilungseinheit (1500) folgende Merkmale aufweist:
einen Arzneimittelverteiler (1510) zum Aufnehmen des gemischten Arzneimittels aus der Arzneimittelmischeinheit (1400); und
Arzneimittelverteilungszweige, die von dem Arzneimittelverteiler (1510) abzweigen, um das gemischte Arzneimittel jeweils für die Mehrzahl von Fischen bereitzustellen.

9. Biosignalmessvorrichtung (1000) gemäß Anspruch 1, bei der die Nadeleinheit (1600) zwischen der Arzneimittelverteilungseinheit (1500) und der Fixiereinheit (1100) angeordnet ist, um der Fixiereinheit (1100) eins zu eins zu entsprechen.

10. Biosignalmessvorrichtung (1000) gemäß Anspruch 9, bei der die Nadeleinheit (1600) folgendes Merkmal aufweist:
einen Nadelhalter, der die Oralnadel fixiert.

11. Biosignalmessvorrichtung (1000) gemäß Anspruch 10, bei der der Nadelhalter eine Höhe und eine Einführtiefe der Oralnadel anpassen kann.

12. Biosignalmessvorrichtung (1000) gemäß Anspruch 1, die ferner eine Erdungseinheit (1700) aufweist, die an der Oralnadel fixiert ist.

13. Biosignalmessvorrichtung (1000) gemäß Anspruch 1, bei der die Elektrodeneinheit (1800) folgende Merkmale aufweist:
Elektrodenanschlussflächen (1811, 1821), die an der Fixiereinheit (1100) fixiert sind; und
Elektroden (1812, 1822), die mit den Elektrodenanschlussflächen (1811, 1821) verbunden sind, um ein Biosignal der Fische zu messen.

## Revendications

1. Dispositif de mesure de signal biologique (1000) destiné à mesurer des signaux biologiques de poisson comprenant :
une unité d'immobilisation (1100) destinée à immobiliser le poisson, dans lequel l'unité d'immobilisation (1100) comprend un logement à poisson (1112) présentant une rainure correspondant à la forme du poisson de telle sorte que le poisson est immobilisé dans celle-ci ;
une unité d'électrode (1800) configurée pour être attachée au poisson afin de mesurer un signal biologique du poisson ; **caractérisé en ce que** le dispositif de mesure de signal biologique comprend en outre :
une unité de stockage de médicaments (1200) destinée à stocker des médicaments à apporter au poisson immobilisé sur l'unité d'immobilisation (1100), dans lequel l'unité de stockage de médicaments (1200) inclut une pluralité d'éléments de stockage de médicaments destinés à stocker différents médicaments, et l'un des éléments de stockage de médicaments contient du liquide contenant de l'oxygène dissous à apporter au poisson de telle sorte que le poisson peut continuer à respirer et à rester en vie lorsqu'un examen est en cours ou même quand il ne l'est pas ;
une unité de mélange de médicaments (1400) destinée à mélanger les médicaments fournis depuis l'unité de stockage de médicaments (1200) ;
une unité de distribution de médicaments (1500) destinée à recevoir les médicaments mélangés provenant de l'unité de mélange de médicaments (1400) et à distribuer les médicaments mélangés à chacun d'une pluralité de poissons ;
une unité d'aiguille (1600) destinée à injecter les médicaments distribués depuis l'unité de distribution de médicaments (1500) à chaque poisson, dans lequel l'unité d'aiguille (1600) comprend une aiguille orale reliée à l'unité de distribution de médicaments (1500) pour recevoir le médicament et configurée pour être insérée dans la bouche du poisson pour injecter les médicaments.

2. Dispositif de mesure de signal biologique (1000) selon la revendication 1, dans lequel l'unité d'immobilisation (1100) comporte :
un corps principal d'immobilisation (1111) formant un corps ; et
un logement à aiguille (1113) formé selon une forme de trou, dans lequel peut être insérée l'aiguille orale, devant le corps principal d'immobilisation (1111) pour communiquer avec le logement à poisson (1112), et
dans lequel le logement à poisson (1112) est formé sur le corps principal d'immobilisation (1111) et le poisson est immobilisé dans une position dressée dans la rainure.

3. Dispositif de mesure de signal biologique (1000) selon la revendication 2, dans lequel l'unité d'immobilisation (1100) comporte en outre des drains (1114) formés au niveau des deux côtés du corps principal d'immobilisation (1111), et
les drains (1114) évacuent les médicaments évacués depuis les branchies du poisson vers l'extérieur depuis le corps principal d'immobilisation (1111).

4. Dispositif de mesure de signal biologique (1000) selon la revendication 2, dans lequel l'unité d'immobilisation (1100) comporte en outre :
une rainure de couplage formée au niveau d'un côté du corps principal d'immobilisation (1111) ; et
une saillie de couplage formée au niveau de l'autre côté du corps principal d'immobilisation (1111) à installer dans la rainure de couplage formée dans un autre corps principal d'immobilisation (1111) adjacent, et
la rainure de couplage et la saillie de couplage maintiennent l'espace entre les corps principaux d'immobilisation adjacents.

5. Dispositif de mesure de signal biologique (1000) selon la revendication 2, dans lequel l'unité d'immobilisation (1100) comporte en outre :
un logement à plot (1117) formé dans le corps principal d'immobilisation (1111) selon une forme de rainure qui peut immobiliser des plots d'électrode (1811, 1821) de l'unité d'électrode (1800) ; et
un trou pour fil (1118) formé dans l'arrière du corps principal d'immobilisation (1111) pour communiquer avec le logement à plot (1117), et
le trou pour fil (1118) est disposé de telle sorte qu'un fil connecté aux plots d'électrode (1811, 1821) immobilisés dans le logement à plot (1117) est inséré dans celui-ci.

6. Dispositif de mesure de signal biologique (1000) selon la revendication 1, comprenant en outre une unité de vanne de médicaments (1300) disposée entre l'unité de stockage de médicaments (1200) et l'unité de mélange de médicaments (1400),
dans lequel l'unité de vanne de médicaments (1300) inclut des vannes de médicaments disposées pour correspondre aux éléments de stockage de médicaments, respectivement, pour commander la quantité de médicaments à apporter à l'unité de mélange de médicaments (1400) depuis les éléments de stockage de médicaments et s'il faut apporter des médicaments.

7. Dispositif de mesure de signal biologique (1000) selon la revendication 1, dans lequel l'unité de mélange de médicaments (1400) inclut :
un échangeur de médicaments (1410) mélangeant les médicaments fournis depuis l'unité de stockage de médicaments (1200) ; et
une pluralité de récepteurs de médicaments disposés pour correspondre aux éléments de stockage de médicaments, respectivement, pour fournir les médicaments dans les éléments de stockage de médicaments à l'échangeur de médicaments (1410).

8. Dispositif de mesure de signal biologique (1000) selon la revendication 1, dans lequel l'unité de distribution de médicaments (1500) inclut :
un distributeur de médicaments (1510) destiné à recevoir le médicament mélangé provenant de l'unité de mélange de médicaments (1400) ; et
des branches de distribution de médicaments divergeant depuis le distributeur de médicaments (1510) pour fournir le médicament mélangé à la pluralité de poissons, respectivement.

9. Dispositif de mesure de signal biologique (1000) selon la revendication 1, dans lequel l'unité d'aiguille (1600) est disposée entre l'unité de distribution de médicaments (1500) et l'unité d'immobilisation (1100) pour correspondre à l'unité d'immobilisation (1100) une à une.

10. Dispositif de mesure de signal biologique (1000) selon la revendication 9, dans lequel l'unité d'aiguille (1600) inclut :
un support d'aiguille immobilisant l'aiguille orale.

11. Dispositif de mesure de signal biologique (1000) selon la revendication 10, dans lequel le support d'aiguille peut ajuster une hauteur et une profondeur d'insertion de l'aiguille orale.

12. Dispositif de mesure de signal biologique (1000) selon la revendication 1, comprenant en outre une unité de terre (1700) fixée à l'aiguille orale.

13. Dispositif de mesure de signal biologique (1000) selon la revendication 1, dans lequel l'unité d'électrode (1800) inclut :
des plots d'électrode (1811, 1821) immobilisés sur l'unité d'immobilisation (1100) ; et
des électrodes (1812, 1822) connectées aux plots d'électrode (1811, 1821) pour mesurer un signal biologique du poisson.
